# EUROPEAN PATENT SPECIFICATION

(11) **EP 1 492 539 B1**
(45) Date of publication and mention of the grant of the patent: **28.06.2006**
(21) Application number: 03745196.0
(22) Date of filing: 28.03.2003
(51) Int. Cl.: A61K 31/55, A61P 25/28

(54) **STATIN THERAPY FOR ENHANCING COGNITIVE MAINTENANCE**
STATIN-THERAPIE ZUR VERBESSERUNG DER AUFRECHTERHALTUNG DER KOGNITIVEN FUNKTION
THERAPIE A BASE DE STATINE PERMETTANT D'AMELIORER L'ENTRETIEN COGNITIF

(30) Priority: 02.04.2002 US 369285 P
(43) Date of publication of application: 05.01.2005
(73) Proprietor: JANSSEN PHARMACEUTICA N.V., 2340 Beerse (BE)
(72) Inventor: LILIENFELD, Sean c/o JANSSEN PHARMACEUTICA Inc., Titusville, NJ 08560 (US); GUTTERMAN, Elane, M., Princeton Junction, NJ 08550 (US); GLASSPOOL, Royston, John, CH-4057 Basel (CH)
(74) Representative: Quaghebeur, Luc
(86) International application number: PCT/EP2003/003324
(87) International publication number: WO 2003/082298

(56) References cited:
- H.JICK E.A.: "Statins and the risk of dementia" THE LANCET, vol. 356, no. 9242, 2000, pages 1627-1631, XP004264153
- M.RAINER, H.MUCKE: "Long-term cognitive benefit from galanthamine in Alzheimer's disease" INTERNATIONAL JOURNAL OF GERIATRIC PSYCHOPHARMACOLOGY, vol. 1, no. 4, 1998, pages 197-201, XP001022883

## Description

### Field of the invention

The present invention relates to a method of treating dementia or a memory disorder comprising administration of a therapeutically effective amount of galantamine (I) and a statin (II). The invention further relates to products containing as first active ingredient galantamine (I) and as second active ingredient a statin (II), as combined preparations for simultaneous, separate or sequential use in the treatment of patients suffering from Alzheimer's disease or related dementias; to related pharmaceutical compositions and uses.

### Background of the invention

Alzheimer's disease (AD) is a chronic neurodegenerative disorder characterized by memory loss and dementia. As in the case of AD, non-Alzheimer's dementias are associated with memory loss and dementia. Both AD and non-AD dementias are also often accompanied by behavioral, psychiatric and/or psychological symptoms including psychosis, depression, anxiety and agitation, and other changes in mood and social withdrawal. In fact, behavioral, psychiatric and/or psychological symptoms of dementia can occur in 60-90% of patients with Alzheimer's disease (AD) or other dementing illnesses, and are critically important since they are the source of significant caregiver stress and can contribute to the caregiver burnout syndrome.

The behavioral, psychiatric and/or psychological clinical manifestations associated with dementia or a memory disorder, more particularly with Alzheimer's disease (AD), can be assessed through clinically accepted scales, for instance, the Brief Psychiatric Rating Scale, the Alzheimer's Disease Assessment Scale-noncognitive, the Relative's Assessment of Global Symptomatology, the Dementia Behavior Disturbance Scale, the Neuropsychiatric Inventory, the Cornell Scale for Depression in Dementia, the Cohen-Mansfield Agitation Inventory, Geriatric Depression Scale, Behavior Rating Scale, Disability Assessment for Dementia, Caregiver time, and the Dementia Mood Assessment Scale.

The treatment of behavioral, psychiatric and/or psychological manifestations in patients with dementia or a memory disorder in the primary care, hospital and nursing home settings includes the use of antipsychotics, antidepressants, anxiolytics and anti-epileptics/anticonvulsants such as carbamazepine and valproic acid.

Acetylcholinesterase (AChE) is an enzyme that plays a pivotal role in cholinergic (acetylcholine) neurotransmission. Physiologically, the hydrolysis of acetylcholine to acetate and choline serves to inactivate acetylcholine molecules released from synaptic terminals and thereby terminate the synaptic signalling event initiated by the release of acetylcholine (ACh) from the nerve terminal. AChE inhibitors are a class of compounds which inhibit the enzyme that degrades acetylcholine. Thus, by inhibiting AChE, the residence time of acetylcholine in the synapsis is prolonged and consequently acetylcholine action is enhanced. Acetylcholinesterase inhibitors include galantamine, rivastigmine, donepezil, and tacrine.

Galantamine, also known as galanthamine or (4aS, 6R, 8aS)-4a, 5, 9, 10, 11, 12-hexahydro-3-methoxy-11-methyl-6H-benzofuro[3a, 3, 2-ef][2]benzazepin-6-ol, is a naturally occurring organic substance, which may be derived from bulbs of the common snowdrop and several Amaryllidaceae plants and which also can be prepared synthetically. More recently, galantamine has been the subject of clinical evaluation for symptomatic treatment of neurological and behavioral signs associated with Alzheimer's Disease, and is currently approved or pending approval for marketing in many world markets under the trade name REMINYL®.

The known pharmacology of galantamine includes an ability to inhibit the AChE. The therapeutic value of an AChE-inhibitor, such as galantamine, derives from the fact that in the brain of AD patients, some of the neurons that release ACh as a synaptic signalling messenger (neurotransmitter) are dysfunctional or non-functional, due to cell death or synaptic degeneration. AChE inhibitors enhance ACh-mediated synaptic activity in this pathological circumstance by prolonging the time that ACh molecules released by the remaining functional synaptic terminals are available to activate ACh receptors in the membrane of the postsynaptic neurons. Galantamine is a reversible cholinesterase inhibitor. Galantamine interacts competitively with the enzyme, acetylcholinesterase, and demonstrates a 10 to 50 fold selectivity for acetyl vs. butyryl cholinesterase.

More recently, new pharmacological properties of galantamine have been discovered which suggests that galantamine may enhance the activity of ACh by mechanisms independent of its ability to inhibit AChE such as allosteric modulation of nicotinic receptors. Galantamine has also been reported to have benefits on the behavioral and psychiatric symptoms of AD.

Galantamine has been used for the treatment of a number of chronic diseases, where life-long treatment may be necessary. Galantamine has been shown to be effective in the treatment of arthritic disorders ; fatigue syndromes; mania ; schizophrenia ; memory dysfunction, including Alzheimer's Disease (US 4,663,318); alcoholism; nicotine dependence; disorders of attention (WO 99/21561) and jet lag

Statins inhibit 3-hydroxy-3-methylglutaryl coenzyme A (HMG-CoA) reductase, the rate limiting enzyme in cholesterol biosynthesis, and are useful in the treatment and prevention of hypercholesterolemia, hyperlipidemia, atherosclerosis and the like.

There is a hypothetized relationship between cholesterol and the production of β-amyloid, a protein that collects in the brains of patients with Alzheimer's disease; statins may reduce the production of β-amyloid. Suggestive findings are:
- Patients taking statins (HMG coenzyme-A reductase inhibitors) have a lower prevalence of probable Alzheimer's disease and dementia
- Cholesterol increases β-amyloid peptide production in the brains of rabbits and mice
- Statins decrease β-amyloid peptide production in brain neurons in culture and in brains of living animals (guinea pigs)
- Patient with high low-density lipoprotein cholesterol show decreased serum β-amyloid when treated with lovastatin 40 or 60 mg in a placebo-controlled, randomized clinical trial.

### Summary of the invention

The present invention relates to a method of treating dementia or a memory disorder comprising administration of a therapeutically effective amount of galantamine (I) and a statin (II). Typically, the dementia is dementia as a result of Alzheimer's Disease (AD. The effect of statins on cognitive maintenance in Alzheimer's disease patients and the safety of coadministering statins and galantamine were studied during 5 to 6 months randomized clinical trial trials of galantamine. The combined use of a statin and galantamine proved to add to the cognitive benefit compared to that obtained with galantamine alone.

The statin (II) is selected from the group comprising simvastatin, pravastatin, lovastatin, fluvastatin, atorvastatin or rosuvastatin, or a therapeutically active acid addition salt form of any of the foregoing. Said salts comprise salt forms which the active ingredients (II) are able to form with appropriate acids, such as, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, *p*-aminosalicylic, pamoic and the like acids. Galantamine (I) may conveniently be used as the (1:1) hydrobromide salt.

Preferably, the amount of statin (II) is equal to or less than that which is approved in monotherapy with said statin (II).

Most preferred are products wherein the amount of galantamine (I) as base is 8, 16 or 24 mg per dosage form.

The present invention also relates to products containing as first active ingredient galantamine (I) and as second active ingredient a statin (II), as combined preparations for simultaneous, separate or sequential use in the treatment of patients suffering from dementia or a memory disorder.

The present invention is also concerned with pharmaceutical compositions comprising a carrier and as first active ingredient galantamine (I) and as second active ingredient a statin (II), preferably, each in an amount producing a therapeutic effect in patients suffering from dementia or a memory disorder. The invention also concerns a process for preparing the aforementioned pharmaceutical compositions.

Further, the present invention also concerns the use of a statin (II) for the preparation of a medicament for enhancing the therapeutic effect of galantamine (I) in patients suffering from dementia or a memory disorder.

### Detailed description of the invention

As used herein, the term "dementia" shall include the deterioration of intellectual and other mental processes, regardless of underlying cause that impairs daily activities and is the result of a deficit in a previously successful performance. Suitable examples of dementia include, but are not limited to, dementia as a result of Alzheimer's disease, vascular related dementia, multi-infarct related dementia, dementia as a result of head trauma, dementia as a result of diffuse brain damage, dementia pugilistica, dementia as a result of Huntington's disease, dementia as a result of alcoholism, dementia as a result of diffuse white matter disease, dementia associated with Parkinson's disease, dementia as a result of Lewy body disease, dementia as a result of Pick's disease, dementia as a result of multisystem degeneration, dementia as a result of progressive supranuclear palsy, dementia associated with the ALS-Parkinson's-Dementia complex of Guam, frontal lobe dementia, and dementia as a result of cortical basal degeneration.

As used herein, the term "memory disorder" shall include memory loss, mental deterioration, diminished mental capacity and loss of cognition.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated. More particularly, in the present invention directed to combination therapy comprising administration of galantamine with one or more statins, "therapeutically effective amount" shall mean that amount of the combination of agents taken together so that the combined effect elicits the desired biological or medicinal response. For example, the therapeutically effective amount of galantamine and simvastatin would be the amount of galantamine and the amount of the simvastatin that when taken together or sequentially have a combined effect that is therapeutically effective. Further, it will be recognized by one skilled in the art that in the case of co-therapy with a therapeutically effective amount, as in the example above, the amount of the galantamine and/or the amount of simvastatin individually may or may not be therapeutically effective.

As used herein, the term "composition" is intended to encompass a product comprising the specified ingredients in the specified amounts, as well as any product which results, directly or indirectly, from combinations of the specified ingredients in the specified amounts.

The individual components of the combination can be administered by any suitable means, simultaneously, sequentially, separately or in a single pharmaceutical formulation. Where galantamine and the statin(s) are administered in separate dosage forms, the number of dosages administered per day for each compound may be the same or different. Galantamine and the statin(s) may be administered via the same or different routes of administration. Examples of suitable methods of administration include, but are not limited to, oral, intravenous (iv), intramuscular (im), subcutaneous (sc), transdermal, and rectal. Compounds may also be administered directly to the nervous system including, but not limited to, intracerebral, intraventricular, intracerebroventricular, intrathecal, intracisternal, intraspinal and / or peri-spinal routes of administration by delivery via intracranial or intravertebral needles and / or catheters with or without pump devices. Galantamine and the statin(s) may be administered according to simultaneous or alternating regimens, at the same or different times during the course of the therapy, concurrently in divided or single forms. The instant invention is therefore to be understood as embacing all such regimens of simultaneous or alternating treatment and the term "administering" is to be interpreted accordingly.

Optimal dosages and dosage regimens to be administered may be readily determined by those skilled in the art, and will vary with the mode of administration, the strength of the preparation and the advancement of the disease condition. In addition, factors associated with the particular patient being treated, including patient's sex, age, weight, diet, physical activity, time of administration and concomitant diseases, will result in the need to adjust dosages and/or regimens.

### Example

The objective was to evaluate the effect of statins on cognitive maintenance in patients with Alzheimer's disease during 5- and 6-month randomized clinical trials of galantamine.

### Study design

- Data were combined from 3 double-blind, placebo-controlled clinical trials limited to patients treated with galantamine 24 mg daily or placebo
- Patients were categorized based on galantamine status and any statin use.

### Efficacy outcomes

- Changes in the Alzheimer's Disease Assessment Scale-cognitive subscale with standard 11 items (ADAS-cog/11) using last observation carried forward (LOCF) were assessed
- Comparisons between patient subgroups were made controlling for relevant confounding factors.

### Safety outcomes

- Rates of adverse side effects commonly linked to acetylcholinesterase inhibitors, including nausea, diarrhea, anorexia, and vomiting, as well as any of these gastrointestinal symptoms were calculated, and the relative risk of galantamine with a statin versus galantamine alone was compared.
- Rates of adverse events commonly linked to statins, including back pain, leg cramps, skeletal pain, muscle atrophy, muscle weakness, and myalgia, as well as any of these muscular-skeletal symptoms were calculated, and the relative risk of galantamine with a statin versus a statin alone was compared.
- Rates of adverse events commonly linked to acetylcholinesterase inhibitors or statins, including abdominal pain or headache were calculated, and the relative risks of galantamine with a statin versus galantamine alone, and versus a statin alone, were compared.

### Limitations

As a concomitant medication, patterns of stain use were heterogeneous in dose, type and duration. The study was not powered for the examination of statin effects.

### Results

### Characteristics of drug treatment groups

Baseline demographics and patient characteristics of each treatment group are summarized in table 1.

**Table 1. Characteristics of drug treatment groups**

| | **STATIN +** | **STATIN** | **GAL 24** | **Neither** | **p-values**^{**1**} |
|---|---|---|---|---|---|
| | **GAL 24** | **only** | **only** | | |
| | n=42 | n=50 | n=614 | n=619 | |
| **Combined trial** | ***Percent*** | ***Percent*** | ***Percent*** | ***Percent*** | |
| Sex, % female | 47.6% | 58.0% | 66.3% | 63.0% | 0.063 |
| | | | | | |

| | ***Mean (SD)*** | ***Mean (SD)*** | ***Mean (SD)*** | ***Mean (SD)*** | |
|---|---|---|---|---|---|
| Age in years ² | 72.4 (8.4) | 74.0 (7.8) | 75.7 (7.9) | 75.2 (8.2) | 0.045 |
| ADAS-Cog baseline | 26.8 (11.3) | 25.7 (8.9) | 26.5 (10.1) | 26.8 (10.6) | 0.852 |
| Total cholesterol ³ | 218.0 (47.2) | 219.3 (38.1) | 228.3 (43.6) | 224.6 (43.8) | 0.177 |
| MMSE baseline⁴ | 18.2(4.1) | 18.4(3.8) | 18.7(3.8) | 18.7(4.0) | 0.784 |

| | ***Percent*** | ***Percent*** | ***Percent*** | ***Percent*** | |
|---|---|---|---|---|---|
| AD severity, % mild⁴ | 61.9% | 66.0% | 64.8% | 64.9% | 0.98 |

| | | | | | |
|---|---|---|---|---|---|
| All values are means (SD) unless otherwise indicated. | | | | | |
| ¹ P-values are based on analysis of variance for continuous variables and chi-square for categorical variables | | | | | |
| ² Pairwise comparisons significant at p ≤ 0.05: Statin +GAL vs GAL only; Statin + GAL vs Neither. | | | | | |
| ³Due to missing data N= 1311. | | | | | |
| ⁴ Mini-Mental State Examination (MMSE): mild (MMSE ≥18) as contrasted to moderate (MMSE < 18). | | | | | |

The proportion of patients taking statins was 6.9% (n = 92).
The statin subgroups had a lower proportion of females (p = 0.06)
The Statin + GAL group was younger by 3 years than non-statin groups (p= 0.05)
The groups were similar on cognitive scores at baseline and total cholesterol.

Distribution of statin type by group are shown in Table 2.

**Table 2 : Distribution of Statin Type in Galantamine Clinical Trial Patients Treated with Statins (n=92)**

| | **STATIN + GAL 24**^{**1**} | | **STATIN only** ^{**2**} | | **TOTAL** | | **P-value** ^{**3**} |
|---|---|---|---|---|---|---|---|
| **Type of Statin** ^{**4**} | *percent* | *(n)* | *percent* | *(n)* | *percent* | *(n)* | |
| atorvastatin | 2.4% | (1) | 18.0% | (9) | 10.9% | (10) | 0.135 |
| fluvastatin | 11.9% | (5) | 8.0% | (4) | 9.8% | (9) | |
| lovastatin | 21.4% | (9) | 24.0% | (12) | 22.8% | (21) | |
| pravastatin | 26.2% | (11) | 16.0% | (19) | 20.7% | (19) | |
| simvastatin | 38.1% | (16) | 34.0% | (17) | 35.9% | (33) | |
| | 100% | (42) | 100% | (50) | 100% | (92) | |

| | | | | | | | |
|---|---|---|---|---|---|---|---|
| ¹ Patients treated with galantamine 24 mg/d and a statin as a concomitant medication | | | | | | | |
| ² Patients treated with placebo and a statin as a concomitant medication | | | | | | | |
| ³ P-value based on chi-square | | | | | | | |
| ⁴ In the 10 patients treated with statins who used 2 types, only first statin used was coded. | | | | | | | |

Patients were treated with 5 different statins (simvastatin, pravastatin, lovastatin, fluvastatin, atorvastatin) with no significant difference in distribution by statin group (p = 0.135).
Simvastatin had the highest frequency of use (38.1% in the Statin + GAL group and 34 % in the Statin-only group).
Statins that produce the greatest reductions in serum low-density lipoprotein cholesterol (simvastatin and atorvastatin) were used by a higher proportion of patients in the Statin-only group (40.5% in the Statin + GAL group and 52% in the Statin-only group).
Statins that penetrate the central nervous system (simvastatin and lovastatin) were used by a similar proportion of patients in both statin groups (59.5 % in the Statin + GAL group and 58% in the Statin-only group).

### Efficacy of treatment with statins and galantamine (intent-to-treat analysis)

Cognitive status improved in the GAL-only (-0.88, SE 0.25) and Statin + GAL (-2.85, SE 0.91) groups.
Cognitive status declined in the placebo-only (2.24, SE 0.24) and Statin-only (1.98, SE 0.85) groups.
The effect of GAL-only was highly significant (p < 0.001), the effect of statins missed significance (p = 0.083), and the interaction of statin and galantamine was not significant (p = 0.183).
The effect of Statin + GAL appeared to be superior to GAL-only (p=.037) in pairwise comparisons with no adjustment for multiple comparisons.
These results were based on ANOVA, controlling for study and AD severity using MMSE; similar results were also found when analyses were limited to observed case data (Table 3).

**Table 3 : Efficacy of Statin Treatment as Reflected by Change in ADAS-Cog in Combined Trial Data**

| **Combined Trial Data** | **STATIN + GAL 24** | **STATIN only** | **GAL 24 only** | **Neither** | **p-values**^{**2**} | |
|---|---|---|---|---|---|---|
| | *LS Means* | *LS Means* | *LS Means* | *LS Means* | | |
| | *(SE)*^{*1*} | *(SE)*^{*1*} | *(SE)*^{*1*} | *(SE)*^{*1*} | | |
| ***Obserned cases*** | n=37 | n=44 | n=503 | n=525 | | |
| ADAS-Cog change score ³ | -2.53 (0.99) | 2.39 (0.93) | -0.61 (0.28) | 2.48 (0.27) | <.001 | drug |
| | | | | | 0.153 | statin |
| | | | | | 0.193 | statin* drug |
| **Intent-to-treat- analysis** | n=42 | n=50 | n=614 | n=619 | | |
| ADAS-Cog change score ⁴ | -2.85 (0.91) | 1.98 (0.85) | -0.88 (0.25) | 2.24 (0.24) | <.001 | drug |
| | | | | | 0.083 | statin |
| | | | | | 0.183 | statin* drug |

| | | | | | | |
|---|---|---|---|---|---|---|
| ¹Least square (LS) means and standard error (SE) | | | | | | |
| ²Based on 4-way analysis of variance with terms for statin (Y/N), drug (GAL/PBO), study, AD severity based on MMSE, and statin*drug | | | | | | |
| ³Also controls for study (p=0.037) and AD severity (p<.001) | | | | | | |
| ⁴Also controls for study (p=0.065) and AD severity (p<.001) | | | | | | |

### Conclusions

Galantamine improved cognitive function in Alzheimer's disease patients during 5- and 6-month clinical trials, while use of statins did not lead to significant improvement when used alone or in combination with galantamine. However, combined use of statin and galantamine did add to the cognitive benefit experienced with galantamine alone. The results further indicate that high statin doses may not be necessary to obtain positive effects when used in combination with galantamine in older adults. Due to small treatment group numbers, adverse event data are inconclusive. The combination of a statin and galantamine may increase the risk of diarrhea, abdominal pain, and muscle or skeletal pain relative to treatment with statin or galantamine alone.

## Claims

1. A product containing as first active ingredient galantamine and as second active ingredient a statin, as a combined preparation for use in the treatment of patients suffering from dementia or a memory disorder.

2. The product of claim 1 wherein the statin is selected from the group comprising simvastatin, pravastatin, lovastatin, fluvastatin, atorvastatin or rosuvastatin, or a therapeutically active acid addition salt form of any of the foregoing, and galantamine is in the form of galantamine hydrobromide (1:1) salt.

3. The product of claim 1 wherein the amount of statin is equal to or less than that which is approved in monotherapy with said statin.

4. The product of claim 1 wherein the amount of galantamine as base is 8, 16 or 24 mg per dosage form.

5. A pharmaceutical composition comprising a carrier and as first active ingredient galantamine and as second active ingredient a statin.

6. The composition of claim 5, comprising a carrier and as first active ingredient galantamine and as second active ingredient a statin, each in an amount producing a therapeutic effect in patients suffering from dementia or a memory disorder.

7. The composition of claim 5 wherein the statin is selected from the group comprising simvastatin, pravastatin, lavastatin, fluvastatin, atorvastatin or rosuvastatin, or a therapeutically active acid addition salt form of any of the foregoing, and galantamine is in the form of galantamine hydrobromide (1:1) salt.

8. The composition of claim 5 wherein the amount of statin is equal to or less than that which is approved in monotherapy with said statin.

9. The composition of claim 5 wherein the amount of galantamine as base is 8, 16 or 24 mg per dosage form.

10. Use of a statin for the preparation of a medicament for enhancing the therapeutic effect of galantamine in patients suffering from dementia or a memory disorder.

11. The use of claim 10 wherein the statin is selected from the group comprising simvastatin, pravastatin, lovastatin, fluvastatin, atorvastatin or rosuvastatin, or a therapeutically active acid addition salt form of any of the foregoing, and galantamine is in the form of galantamine hydrobromide (1:1) salt.

12. The use of claim 10 wherein the amount of statin is equal to or less than that which is approved in monotherapy with said statin.

13. The use of claim 10 wherein galantamine is also used in the preparation of the medicament.

14. The use of claim 13 wherein the amount of galantamine as base is 8, 16 or 24 mg per dosage form.

15. A process for making a pharmaceutical composition as defined in any of claims 5 to 9 comprising mixing galantamine (I), a statin (II) and a pharmaceutically acceptable carrier.

## Patentansprüche

1. Produkt, das Galantamin als ersten Wirkstoff und ein Statin als zweiten Wirkstoff enthält, als Kombinationspräparat zur Verwendung in der Behandlung von an Demenz oder einer Gedächtnisstörung leidenden Patienten.

2. Produkt nach Anspruch 1, worin das Statin aus der Simvastatin, Pravastatin, Lovastatin, Fluvastatin, Atorvastatin oder Rosuvastatin umfassenden Gruppe ausgewählt ist oder ein therapeutisch wirksames Säureadditionssalz einer der vorgenannten Wirkstoffe ist und Galantamin in Form von Galantaminhydrobromid (1:1) Salz vorliegt.

3. Produkt nach Anspruch 1, worin die Statinmenge gleich oder kleiner ist als die Menge, die in der Monotherapie mit diesem Statin zugelassen ist.

4. Produkt nach Anspruch 1, worin die Menge an Galantamin als Grundlage 8, 16 oder 24 mg pro Dosierform beträgt.

5. Pharmazeutische Zusammensetzung, die einen Träger und Galantamin als ersten Wirkstoff und ein Statin als zweiten Wirkstoff umfasst.

6. Zusammensetzung nach Anspruch 5, die einen Träger und Galantamin als ersten Wirkstoff und ein Statin als zweiten Wirkstoff jeweils in einer Menge umfasst, die bei an Demenz oder einer Gedächtnisstörung leidenden Patienten eine therapeutische Wirkung hervorruft.

7. Zusammensetzung nach Anspruch 5, worin das Statin aus der Simvastatin, Pravastatin, Lovastatin, Fluvastatin, Atorvastatin oder Rosuvastatin umfassenden Gruppe ausgewählt ist oder ein therapeutisch wirksames Säureadditionssalz einer der vorgenannten Wirkstoffe ist und Galantamin in Form von Galantaminhydrobromid (1:1) Salz vorliegt.

8. Zusammensetzung nach Anspruch 5, worin die Statinmenge gleich oder kleiner ist als die Menge, die in der Monotherapie mit diesem Statin zugelassen ist.

9. Zusammensetzung nach Anspruch 5, worin die Menge an Galantamin als Grundlage 8, 16 oder 24 mg pro Dosierform beträgt.

10. Verwendung eines Statins zur Herstellung eines Medikaments zur Verbesserung der therapeutischen Wirkung von Galantamin bei an Demenz oder einer Gedächtnisstörung leidenden Patienten.

11. Verwendung nach Anspruch 10, worin das Statin aus der Simvastatin, Pravastatin, Lovastatin, Fluvastatin, Atorvastatin oder Rosuvastatin umfassenden Gruppe ausgewählt ist oder ein therapeutisch wirksames Säureadditionssalz einer der vorgenannten Wirkstoffe ist und Galantamin in Form von Galantaminhydrobromid (1:1) Salz vorliegt.

12. Verwendung nach Anspruch 10, worin die Statinmenge gleich oder kleiner ist als die Menge, die in der Monotherapie mit diesem Statin zugelassen ist.

13. Verwendung nach Anspruch 10, worin Galantamin auch bei der Herstellung des Medikaments verwendet wird.

14. Verwendung nach Anspruch 13, worin die Menge an Galantamin als Grundlage 8, 16 oder 24 mg pro Dosierform beträgt.

15. Verfahren zur Herstellung einer pharmazeutischen Zusammensetzung wie in einem der Ansprüche 5 bis 9 definiert, bei dem Galantamin (I), ein Statin (II) und ein pharmazeutisch annehmbarer Träger miteinander gemischt werden.

## Revendications

1. Produit contenant, comme premier ingrédient actif, de la galantamine et, comme deuxième ingrédient actif, une statine en tant que préparation combinée destinée à être utilisée dans le traitement de patients souffrant de démence ou d'un trouble de la mémoire.

2. Produit selon la revendication 1, dans lequel la statine est choisie dans le groupe comprenant : la simvastatine ; la pravastatine ; la lovastatine ; la fluvastatine ; l'atorvastatine ; ou la rosuvastatine ; ou une forme de sel d'addition d'acide, actif d'un point de vue thérapeutique, de l'un quelconque des éléments précédents ; et la galantamine se trouve sous forme de sel de bromhydrate de galantamine (1 : 1).

3. Produit selon la revendication 1, dans lequel la quantité de statine est inférieure ou égale à celle qui est approuvée dans une monothérapie avec ladite statine.

4. Produit selon la revendication 1, dans lequel la quantité de galantamine, en tant que base, est de 8, 16 ou 24 mg par forme de dose.

5. Composition pharmaceutique comprenant un véhicule ainsi que, comme premier ingrédient actif, de la galantamine et, comme deuxième ingrédient actif, une statine.

6. Composition, selon la revendication 5, comprenant un véhicule ainsi que, comme premier ingrédient actif, de la galantamine et, comme deuxième ingrédient actif, une statine, chacun d'entre eux en une quantité qui produit un effet thérapeutique chez des patients souffrant de démence ou d'un trouble de la mémoire.

7. Composition selon la revendication 5, dans laquelle la statine est choisie dans le groupe comprenant : la simvastatine ; la pravastatine ; la lovastatine ; la fluvastatine ; l'atorvastatine ; ou la rosuvastatine ; ou une forme de sel d'addition d'acide, actif d'un point de vue thérapeutique, de l'un quelconque des éléments précédents ; et la galantamine se trouve sous forme de sel de bromhydrate de galantamine (1 : 1).

8. Composition selon la revendication 5, dans laquelle la quantité de statine est inférieure ou égale à celle qui est approuvée dans une monothérapie avec ladite statine.

9. Composition selon la revendication 5, dans laquelle la quantité de galantamine, en tant que base, est de 8, 16 ou 24 mg par forme de dose.

10. Utilisation d'une statine pour la préparation d'un médicament destiné à renforcer l'effet thérapeutique de la galantamine chez des patients souffrant de démence ou d'un trouble de la mémoire.

11. Utilisation selon la revendication 10, dans laquelle la statine est choisie dans le groupe comprenant : la simvastatine ; la pravastatine ; la lovastatine ; la fluvastatine ; l'atorvastatine ; ou la rosuvastatine ; ou une forme de sel d'addition d'acide, actif d'un point de vue thérapeutique, de l'un quelconque des éléments précédents ; et la galantamine se trouve sous forme de sel de bromhydrate de galantamine (1 : 1).

12. Utilisation selon la revendication 10, dans laquelle la quantité de statine est inférieure ou égale à celle qui est approuvée dans une monothérapie avec ladite statine.

13. Utilisation, selon la revendication 10, dans laquelle de la galantamine est également utilisée dans la préparation du médicament.

14. Utilisation selon la revendication 13, dans laquelle la quantité de galantamine, en tant que base, est de 8, 16 ou 24 mg par forme de dose.

15. Processus pour élaborer une composition pharmaceutique, selon ce qui est défini dans l'une quelconque des revendications 5 à 9, comprenant l'étape consistant à mélanger la galantamine (I), une statine (II) et un véhicule acceptable d'un point de vue pharmaceutique.
